(19) Europäisches Patentamt
European Patent Office
Office européen des brevets

(11) **EP 4 233 862 A1**

(12) **EUROPEAN PATENT APPLICATION**
published in accordance with Art. 153(4) EPC

(43) Date of publication:
**30.08.2023 Bulletin 2023/35**

(21) Application number: **20964683.5**

(22) Date of filing: **10.12.2020**

(51) International Patent Classification (IPC):
*A61K 31/4168* (2006.01)    *A61K 31/137* (2006.01)
*A61K 9/28* (2006.01)    *A61K 47/38* (2006.01)
*A61K 47/26* (2006.01)    *A61K 47/04* (2006.01)
*A61P 25/36* (2006.01)

(86) International application number:
**PCT/CN2020/135368**

(87) International publication number:
**WO 2022/120745 (16.06.2022 Gazette 2022/24)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.12.2020 CN 202011431216**

(71) Applicant: **Shenzhen Sciencare Pharmaceutical
Co., Ltd.
Shenzhen Guangdong 518000 (CN)**

(72) Inventors:
• **LIANG, Zhenglin**
  **Shenzhen, Guangdong 518000 (CN)**
• **YING, Shugui**
  **Shenzhen, Guangdong 518000 (CN)**
• **YAN, Xieguo**
  **Shenzhen, Guangdong 518000 (CN)**
• **WANG, Shiqiang**
  **Shenzhen, Guangdong 518000 (CN)**
• **ZHANG, Tao**
  **Shenzhen, Guangdong 518000 (CN)**

(74) Representative: **karo IP
karo IP Patentanwälte
Kahlhöfer Rößler Kreuels PartG mbB
Postfach 32 01 02
40416 Düsseldorf (DE)**

(54) **MEDICINE FOR RELIEVING OR ELIMINATING PROTRACTED OPIOID ABSTINENCE SYNDROME AND PREPARATION METHOD THEREFOR**

(57)    The present disclosure provides a pharmaceutical composition for relieving or eliminating opioid withdrawal syndrome and a preparation method therefor. The pharmaceutical composition includes clonidine hydrochloride and tramadol hydrochloride. A compound tablet of the pharmaceutical composition is made of clonidine hydrochloride, tramadol hydrochloride, lactose hydrous, microcrystalline cellulose, silicon dioxide and magnesium stearate. The preparation method includes the following steps: (1) dissolving a formulated amount of clonidine hydrochloride in water, granulating with a formulated amount of microcrystalline cellulose, sieving, and oven-drying to obtain granules; and (2) mixing the granules obtained at step (1) with a formulated amount of tramadol hydrochloride and a formulated amount of lactose hydrate, adding a formulated amount of silicon dioxide and a formulated amount of magnesium stearate, mixing, compressing into tablets, and coating to obtain compound tablets.

EP 4 233 862 A1

**Description**

<u>TECHNICAL FIELD</u>

The present disclosure relates to the technical fields of addiction medicine and neurobiology, and specifically to a medicine for relieving or eliminating opioid withdrawal syndrome and a preparation method therefor .

<u>Background</u>

Drug abuse addiction has become a public hazard in the world. It not only endangers the physical and mental health of addicts, but also seriously interferes with social security and productivity development. Currently, comprehensive measures of medication and psychosocial interventions are recommended for treatment of opioid dependence, including cessation of drug abuse, detoxification treatment of withdrawal symptoms, rehabilitation and anti-relapse treatment of psychological dependence and other physical, psychological, and social function impairments, in an effort to finally help drug addicts recover from addition and reintegrate into society. During treatment, the severity of drug dependence needs to be determined first according to the type, dose, time, and route of an abuse drug, and historical drug withdrawal conditions, and a drug for drug withdrawal and a treatment method are selected according to individual conditions of a drug addict.

Existing treatment measures mainly include: 1. replacement treatment such as replacement treatment with methadone and replacement treatment with buprenorphine; 2. non-replacement treatment such as treatment with clonidine and lofexidine; 3. detoxification treatment with traditional Chinese herbs, there are nearly 10 traditional Chinese herbs approved by National Medical Products Administration for detoxification, which are suitable for drug addicts with mild and moderate opioid dependence, the efficacy of traditional Chinese herbs alone for drug addicts with severe dependence is not satisfactory, and they need to be used in combination with other drugs; and 4. other detoxification treatments, such as acupuncture and electroacupuncture, need to be further verified.

Detoxification is the first phase of the treatment of opioid addiction, and an opioid replacement decreasing method is mainly adopted at this phase. There are only three drugs approved by Food and Drug Administration for the treatment of opioid dependence: methadone, buprenorphine, and naltrexone. Although these drugs can be effective in inhibiting withdrawal symptoms, they have similar dependence-producing potential and can also cause addiction after long-term use.

Clonidine is an centrally-acting $\alpha_2$-adrenergic agonist, stimulates $\alpha_2$-adrenergic receptor, and inhibits the discharge of locus coeruleus in central nervous system, reduce the release of norepinephrine, relieve symptoms, and reduce nausea, vomiting, diarrhea and abdominal pain, and sweating. Compared with methadone, clonidine does not cause rebound of symptoms after drug withdrawal, and can be used as an anti-relapse medication in a transition from detoxification treatment with methadone to maintenance with naltrexone. In the guidelines developed by Ministry of Health for commonly used detoxification treatment of opioid addiction, clonidine is the only one non-opioid therapeutic drug. Therefore, clonidine is commonly used as a control drug in clinic trials of novel non-opioid detoxification drug.

In recent years, clonidine and naltrexone are often used for rapid detoxification. Tramadol is an opioid agonist with an opioid moiety and a non-opioid moiety in its structure. Therefore, in addition to acting through the opioid receptors, tramadol can inhibit the activity of norepinephrine or serotonin in central nervous system, which may be the main mechanism of eliminating opioid withdrawal symptoms by tramadol.

CN103495172A discloses a medicament for treating amphetamine type stimulant dependence and mixed dependence of amphetamine type stimulants and opiates substances. The medicament is composed of 2-95% (percentage by weight) of antipsychotics, 0.001-5% of $\alpha_2$-adrenergic agonist, 0-5% of anticholinergics, 0-80% of non-opioid analgesic, and 0-10% of benzodiazepines. The $\alpha_2$-adrenergic agonist is one or more of clofenoxazoline, chloracetamide, clonidine, and lofexidine, and the non-opioid analgesic is one or more of tetrahydropalmatine, tramadol, nefopam, carbamazepine, rotundine, tetrazepine, and tetrahydropalmatine. The pharmaceutical composition of this disclosure achieves a satisfactory effect when used for treating the foregoing symptoms of a patient with amphetamine type stimulant abuse or combined amphetamine type stimulant and opioid substance abuse, and can quickly and significantly control withdrawal symptoms, with a detoxification recovery rate of greater than 90%. However, this disclosure requires long withdrawal time, the medication compliance and medication experience of the patient are poor, and effects of clinical application are poor.

<u>Summary</u>

The present disclosure provides a medicine for relieving or eliminating opioid withdrawal syndrome and application in order to solve the foregoing technical problems. The pharmaceutical composition is more suitable for detoxification of patients with opioid abuse within a short time period of 3 to 5 days, which is safe and effective, easy to operate, has significant clinical advantages and efficacy, and can improve the medication compliance and medication experience of patients to avoid abuse.

The present disclosure adopts the following technical solutions in order to achieve the foregoing objectives.

The present disclosure provides a pharmaceutical composition for relieving or eliminating opioid withdrawal syndrome, which includes clonidine hydrochloride and tramadol hydrochloride.

Preferably, a mass ratio of clonidine hydrochloride to tramadol hydrochloride is (0.01-0.8): (40-60), and further preferably, (0.01-0.56): 50.

Preferably, a total mass fraction of clonidine hydrochloride and tramadol hydrochloride in the pharmaceutical composition is 15-20%, and further preferably, 17-19%.

The present disclosure also provides application of the foregoing pharmaceutical composition in preparation of a medicine for relieving or eliminating opioid withdrawal syndrome.

The present disclosure also provides a medicament, which is composed of the foregoing pharmaceutical composition for relieving or eliminating opioid withdrawal syndrome and pharmaceutically acceptable excipients.

The medicament includes, but is not limited to, compound tablet, pill, granules, injection, powder, spray, sublingual tablet, capsule, and liquid preparation.

The present disclosure also provides a compound tablet for relieving or eliminating opioid withdrawal syndrome, which includes clonidine hydrochloride, tramadol hydrochloride, lactose hydrate, microcrystalline cellulose, silicon dioxide, and magnesium stearate.

A usage amount of clonidine hydrochloride is 0.01-0.8 parts by weight of the compound tablet.

A usage amount of tramadol hydrochloride is 40-60 parts by weight of the compound tablet.

A usage amount of lactose hydrate is 110-120 parts by weight of the compound tablet.

A usage amount of microcrystalline cellulose is 100-120 parts by weight of the compound tablet.

A usage amount of silicon dioxide is 0.5-1 part by weight of the compound tablet.

A usage amount of magnesium stearate is 2.0-3.6 parts by weight of the compound tablet.

Preferably, the compound tablet includes the following components in parts by weight: 0.01-0.8 part of clonidine hydrochloride, 40-60 parts of tramadol hydrochloride, 110-120 parts of lactose hydrate, 100-120 parts of microcrystalline cellulose, 0.5-1 part of silicon dioxide, and 2.0-3.6 parts of magnesium stearate.

All the foregoing technical solutions can achieve the technical effects described in the present disclosure, and in some preferred embodiments, the technical effects achieved by the foregoing technical solutions are superior to those achieved by other solutions.

A mass ratio of clonidine hydrochloride to tramadol hydrochloride is (0.01-0.56): 50. In a preferred embodiment, the compound tablet includes the following components in parts by weight: 0.01-0.56 part of clonidine hydrochloride, 50 parts of tramadol hydrochloride, 114.5 parts of lactose hydrate, 112 parts of microcrystalline cellulose, 0.7 part of silicon dioxide, and 2.8 parts of magnesium stearate.

The present disclosure also provides a preparation method of a compound tablet for relieving or eliminating opioid withdrawal syndrome, which includes the following steps:

(1) dissolving a formulated amount of clonidine hydrochloride in water, granulating with a formulated amount of microcrystalline cellulose, sieving, and oven-drying to obtain granules; and

(2) mixing the granules obtained at step (1) with a formulated amount of tramadol hydrochloride and a formulated amount of lactose hydrate, adding a formulated amount of silicon dioxide and a formulated amount of magnesium stearate, mixing, compressing into tablets, and coating to obtain compound tablets.

The present disclosure also provides application of the foregoing medicament in preparation of a medicine for relieving or eliminating opioid withdrawal syndrome.

Compared with the prior art, the present disclosure has the following advantages and effects.

(1) The present disclosure is more suitable for detoxification of patients with opioid abuse within a short time period of 3 to 5 days, which is safe and effective, easy to operate, has significant clinical advantages and efficacy, and can improve the medication compliance and medication experience of patients to avoid abuse.

(2) Through the synergistic compounding of clonidine hydrochloride and tramadol hydrochloride, the pharmaceutical composition of the present disclosure has a good effect of relieving or eliminating withdrawal symptoms of mice with morphine dependence, and has a low risk of addiction and good safety.

(3) The preparation method of the present disclosure is simple and low in cost, and prepared compound tablets have good stability.

**Detailed Description of the Embodiments**

To make the objectives, technical solutions, and advantages of the present disclosure clearer, the technical solutions of the present disclosure will be described in details below.

It should be understood that, when a numerical range is given in an example, unless otherwise described, two endpoints of each numerical range and any one numerical value between the two endpoints can be selected. Unless otherwise defined, all technical and scientific terms used herein have the same meaning as commonly understood by those of ordinary skill in the art to which the present disclosure belongs.

Unless otherwise described, all raw materials used in the present disclosure are general commercially available products.

Examples 1 to 3 Compound tablets for relieving or eliminating opioid withdrawal syndrome

Formulations of the compound tablets for relieving or eliminating opioid withdrawal syndrome of Examples 1 to 3 are shown in Table 1.

Table 1 Formulations of compound tablets for alleviating or eliminating opioid withdrawal syndrome (each tablet containing /mg)

| Raw material | Example 1 | Example 2 | Example 3 |
|---|---|---|---|
| Clonidine hydrochloride | 0.6 | 0.7 | 0.8 |
| Tramadol hydrochloride | 40 | 50 | 60 |
| Lactose monohydrate | 110 | 115 | 120 |
| Microcrystalline cellulose | 110 | 112 | 120 |
| Silicon dioxide | 0.5 | 0.7 | 1 |
| Magnesium stearate | 2 | 3 | 3.6 |
| Total | 263.1 | 281.4 | 305.4 |

Examples 4 to 10 Compound tablets for relieving or eliminating opioid withdrawal syndrome

Formulations of the compound tablets for relieving or eliminating opioid withdrawal syndrome of Examples 4 to 10 are shown in Table 2.

Table 2 Formulations of compound tablets for alleviating or eliminating opioid withdrawal syndrome (each tablet containing / mg)

| Raw material | Examp le 4 | Examp le 5 | Examp le 6 | Examp le 7 | Examp le 8 | Examp le 9 | Examp le 10 |
|---|---|---|---|---|---|---|---|
| Clonidine hydrochloride | 0.01 | 0.02 | 0.05 | 0.1 | 0.2 | 0.25 | 0.56 |
| Tramadol hydrochloride | 50 | 50 | 50 | 50 | 50 | 50 | 50 |
| Lactose monohydrate | 114.5 | 114.5 | 114.5 | 114.5 | 114.5 | 114.5 | 114.5 |
| Microcrystalli ne cellulose | 112 | 112 | 112 | 112 | 112 | 112 | 112 |
| Silicon dioxide | 0.7 | 0.7 | 0.7 | 0.7 | 0.7 | 0.7 | 0.7 |
| Magnesium stearate | 2.8 | 2.8 | 2.8 | 2.8 | 2.8 | 2.8 | 2.8 |
| Total | 280.01 | 280.02 | 280.05 | 280.1 | 280.2 | 280.25 | 280.56 |

A preparation method of the compound tablets included the following steps:

(1) a formulated amount of clonidine hydrochloride was dissolved in water, the solution was prepared into granules with a formulated amount of microcrystalline cellulose, and the granules were sieved and oven-dried; and

(2) the granules obtained at step (1) were mixed with a formulated amount of tramadol hydrochloride and a formulated amount of lactose monohydrate, a formulated amount of silicon dioxide and a formulated amount of magnesium stearate were added, and the mixture was mixed, compressed into tablets, and coated to obtain compound tablets.

Comparative Example 1

A different between this Comparative Example and Example 10 was that clonidine hydrochloride was not included.

Comparative Example 2

A difference between this Comparative Example and Example 10 was that tramadol hydrochloride was not included.

Experimental Example I Stability

50 compound tablets of each of Examples 1 to 10 were taken and placed at the temperature of 40±2°C and the relative humidity of 75±5% for 6 months, and the tablets were taken out for determination of content and related substance after 1 month, 2 months, 3 months, and 6 months.

(1) The content was measured by high-performance liquid chromatography (see General Rule 0512 in *Chinese Pharmacopoeia* 2020), which was specifically as follows.

A test solution 1: 15-20 compound tablets were taken, accurately weighed, and finely ground, an appropriate amount (equivalent to 50 mg of tramadol hydrochloride) of ground tablets were taken, accurately weighed, and placed into a 100 mL volumetric flask, an appropriate amount of mobile phase was added, the ground tablets were dissolved by an ultrasonic method, the solution was placed until cool, a mobile phase was added to dilute the solution to a scale, the mixture was uniformly shaken and filtered, and a subsequent filtrate was taken as a test solution 1.

A test solution 2: 15-20 compound tablets were taken, accurately weighed, and finely ground, an appropriate amount (approximately equivalent to 0.4 mg of clonidine hydrochloride) of ground tablets were accurately weighed and placed into a 100 mL volumetric flask, an appropriate amount of mobile phase was added, the ground tablets were dissolved by an ultrasonic method, the solution was placed until cool, a mobile phase was added to dissolve and dilute the solution to a scale, the mixture was uniformly shaken and filtered, and a subsequent filtrate was taken as a test solution 2.

Clonidine reference stock solution: 20 mg of clonidine hydrochloride reference was taken, accurately weighed, and placed into a 100 mL volumetric flask, a blank solution was added to dissolve the clonidine hydrochloride reference and dilute to a scale, the solution was uniformly shaken and taken as a clonidine reference stock solution.

Reference solution: 25 mg of tramadol hydrochloride was taken, accurately weighed, and placed into a 50 mL volumetric flask, an appropriate amount of blank solution was added to dissolve the tramadol hydrochloride, 1.0 mL of clonidine hydrochloride reference stock solution was accurately added, a blank solution was added to dissolve dilute the mixed solution to a scale, and the mixture was uniformly shaken and taken as a reference solution.

Chromatographic conditions: see related substances below.

According to requirements of system suitability, the number of theoretical plates shall be not less than 1500 based on a tramadol peak.

Measurement method: the test solution and the reference solution were respectively accurately weighed and injected into a liquid chromatography instrument. The number of theoretical plates was calculated based on a peak area by an external standard method.

A calculation formula is as follows:

$$\text{content\%} = \frac{F \times A \times V \times M}{m \times Mr \times 1000} \times 100\%;$$

where, m is the weight of a test sample in a unit of mg;

V is the diluted volume of a reference solution in a unit of mL;

A is the peak area of a test solution;

F is a calculation factor;

M is the average tablet weight in a unit of mg; and

Mr is the labelled weight in a unit of mg.

(2) Related substances were detected by high-performance liquid chromatography (see General 0512 in *Chinese Pharmacopoeia* 2020), which was specifically as follows.

Test solution: an appropriate amount of fine powder was taken and accurately weighed, a mobile phase was added to dissolve the fine powder and quantitatively dilute so as to obtain a solution containing 2 mg/mL tramadol hydrochloride, the solution was uniformly shaken and filtered, and a subsequent filtrate was taken as a test solution.

Tramadol impurity I reference solution: an appropriate amount of tramadol impurity I reference was taken and accurately weighed, and a mobile phase was added to dissolve the tramadol impurity I reference and quantitatively dilute so as to obtain a solution containing 0.6 mg/mL tramadol impurity I reference.

Clonidine hydrochloride reference stock solution: an appropriate amount of clonidine hydrochloride reference was taken and accurately weighed, and a blank solution was added to dissolve the clonidine hydrochloride reference and dilute so as to obtain a solution containing 0.2 mg/mL clonidine hydrochloride reference.

System suitability solution: 1 mL of test solution, 2 mL of clonidine hydrochloride reference stock solution, and 1 mL of tramadol impurity I reference solution were respectively accurately weighed and placed into a 100 mL volumetric flask, a mobile phase was added for dissolution and dilution to a scale, and the mixture was uniformly shaken and taken as a system suitability solution.

Reference solution: 1 mL of test solution was accurately weighed and placed into a 100 mL volumetric flask, a mobile phase was added for dissolution and dilution to a scale, and the mixture was shaken and taken as a reference solution.

Chromatographic conditions: octadecyl silane bonded silica gel was used as a filler (recommended chromatographic column: Welch C18 4.6*250 nm, 5 μm); acetic acid sodium acetate buffer (pH=4.5) (18 g of sodium acetate trihydrate was taken, 9.8 mL of glacial acetic acid was added, and water was added for dilution to 1000 mL)-methanol (65: 35) was used as a mobile phase; a detection wavelength was 271 nm; and the injection volume was 20 uL.

According to requirements of system suitability, the number of theoretical plates shall be not less than 1500 based on a tramadol peak. A peaking sequence is: clonidine, tramadol impurity I, and tramadol. The separation between the impurity I peak and the tramadol peak shall be greater than 2.0.

Measurement method: the test solution and the reference solution were respectively accurately weighed and injected into a liquid chromatography instrument until 3 times the retention time of the principal component peak.

Limitation: if the test solution has impurity peaks, the impurity I peak area shall not be greater than 0.3 time (0.3%) the reference main peak area, and the sum of the remaining impurity peak areas shall not be greater than the reference main peak area (1.0%).

Test results are shown in Table 3.

Table 3

| | Condition | Impurity I (%) | Maximum single impurity (%) | Total impurity % (except the impurity I) | Number of impurities | Tramadol content (%) | Clonidine content (%) |
|---|---|---|---|---|---|---|---|
| Example 1 | 0 day | 0.01 | 0.03 | 0.06 | 5 | 102.2 | 97.1 |
| | 1 month | 0.01 | 0.02 | 0.05 | 5 | 100.6 | 94.2 |
| | 2 months | 0.01 | 0.03 | 0.06 | 5 | 98.0 | 92.0 |
| | 3 months | 0.02 | 0.03 | 0.06 | 5 | 97.6 | 91.9 |
| | 6 months | 0.03 | 0.03 | 0.05 | 5 | 95.6 | 89.7 |
| Example 2 | 0 day | 0.01 | 0.02 | 0.05 | 5 | 100.3 | 100.2 |
| | 1 month | 0.01 | 0.02 | 0.06 | 5 | 100.1 | 99.7 |
| | 2 months | 0.01 | 0.03 | 0.05 | 5 | 99.9 | 98.0 |
| | 3 months | 0.02 | 0.03 | 0.06 | 5 | 97.0 | 95.0 |
| | 6 months | 0.03 | 0.03 | 0.06 | 5 | 96.0 | 89.6 |

(continued)

|  | Conditio n | Impuri ty I (%) | Maximu m single impurity (%) | Total impurity % (except the impurity I) | Number of impuriti es | Tramad ol content (%) | Clonidin e content (%) |
|---|---|---|---|---|---|---|---|
| Exampl e 3 | 0 day | 0.01 | 0.03 | 0.05 | 5 | 97.8 | 96.7 |
|  | 1 month | 0.01 | 0.02 | 0.06 | 5 | 97.6 | 95.6 |
|  | 2 months | 0.01 | 0.03 | 0.06 | 5 | 96.3 | 93.1 |
|  | 3 months | 0.03 | 0.02 | 0.06 | 5 | 95.8 | 92.0 |
|  | 6 months | 0.03 | 0.03 | 0.06 | 5 | 95.7 | 89.8 |
| Exampl e4 | 0 day | 0.01 | 0.03 | 0.05 | 4 | 101.4 | 99.9 |
|  | 1 month | 0.01 | 0.03 | 0.05 | 4 | 100.9 | 98.8 |
|  | 2 months | 0.01 | 0.03 | 0.04 | 4 | 100.7 | 94.4 |
|  | 3 months | 0.01 | 0.03 | 0.05 | 4 | 100.5 | 94.3 |
|  | 6 months | 0.01 | 0.03 | 0.05 | 4 | 100.2 | 92.7 |
| Exampl e5 | 0 day | 0.01 | 0.03 | 0.05 | 4 | 101.4 | 99.9 |
|  | 1 month | 0.01 | 0.03 | 0.05 | 4 | 100.9 | 98.8 |
|  | 2 months | 0.01 | 0.03 | 0.04 | 4 | 100.7 | 94.4 |
|  | 3 months | 0.01 | 0.03 | 0.05 | 4 | 100.5 | 94.1 |
|  | 6 months | 0.01 | 0.03 | 0.05 | 4 | 100.3 | 93.9 |
| Exampl e6 | 0 day | 0.01 | 0.03 | 0.05 | 4 | 101.4 | 99.9 |
|  | 1 month | 0.01 | 0.03 | 0.05 | 4 | 100.9 | 98.8 |
|  | 2 months | 0.01 | 0.03 | 0.04 | 4 | 100.7 | 94.4 |
|  | 3 months | 0.01 | 0.02 | 0.05 | 4 | 100.6 | 94.0 |
|  | 6 months | 0.01 | 0.03 | 0.04 | 4 | 100.5 | 93.8 |
| Exampl e7 | 0 day | 0.01 | 0.03 | 0.05 | 4 | 100.1 | 98.9 |
|  | 1 month | 0.01 | 0.03 | 0.05 | 4 | 101.7 | 99.0 |
|  | 2 months | 0.01 | 0.02 | 0.05 | 4 | 98.8 | 96.0 |
|  | 3 months | 0.01 | 0.03 | 0.05 | 4 | 99.7 | 93.6 |
|  | 6 months | 0.01 | 0.03 | 0.05 | 4 | 99.0 | 92.7 |
| Exampl e8 | 0 day | 0.01 | 0.03 | 0.05 | 4 | 102.2 | 97.1 |
|  | 1 month | 0.01 | 0.02 | 0.04 | 4 | 100.6 | 94.2 |
|  | 2 months | 0.01 | 0.03 | 0.05 | 4 | 98.0 | 92.0 |
|  | 3 months | 0.01 | 0.03 | 0.05 | 4 | 100.6 | 91.9 |
|  | 6 months | 0.01 | 0.03 | 0.05 | 4 | 99.7 | 91.7 |
| Exampl e9 | 0 day | 0.01 | 0.02 | 0.04 | 4 | 100.3 | 100.2 |
|  | 1 month | 0.01 | 0.02 | 0.05 | 4 | 100.1 | 99.7 |
|  | 2 months | 0.01 | 0.03 | 0.04 | 4 | 99.9 | 98.0 |
|  | 3 months | 0.01 | 0.03 | 0.05 | 4 | 99.8 | 96.0 |
|  | 6 months | 0.01 | 0.03 | 0.05 | 4 | 99.7 | 92.3 |

(continued)

| | Condition | Impurity I (%) | Maximum single impurity (%) | Total impurity % (except the impurity I) | Number of impurities | Tramadol content (%) | Clonidine content (%) |
|---|---|---|---|---|---|---|---|
| Example 10 | 0 day | 0.01 | 0.03 | 0.04 | 4 | 97.8 | 96.7 |
| | 1 month | 0.01 | 0.02 | 0.05 | 4 | 97.6 | 96.0 |
| | 2 months | 0.01 | 0.03 | 0.05 | 4 | 97.7 | 93.1 |
| | 3 months | 0.01 | 0.02 | 0.05 | 4 | 97.6 | 92.3 |
| | 6 months | 0.01 | 0.03 | 0.05 | 4 | 96.9 | 92.0 |

It can be seen from the foregoing table that the compound tablets of the present disclosure have good stability.

Experimental Example II Main pharmaceutical effects of drugs in inhibiting morphine withdrawal symptoms

(1) Mouse jumping test

Mice were randomly divided into a blank control group and drug groups 1 to 12 according to the weight. The drug groups 1 to 12 (respectively administered with the compound tablets of Example 1 to 10 and the tablets of Comparative Examples 1 and 2) were divided into low-dose groups, medium-dose groups, and high-dose groups, 10 mice per group. The mice in each group were intraperitoneally injected with morphine 6 times a day at administration doses of 10 mg/kg/time on day 1 and 10 mg/kg/time on day 2 and day 3. 50 minutes after the last injection of morphine, the mice in the low-dose drug groups, the medium-dose drug groups, and the high-dose drug groups were respectively subjected to intragastric administration at doses of 36 mg/kg, 72 mg/kg, and 143 mg/kg, and the mice in the blank control group were administered with an equal volume of normal saline. 30 minutes after intragastric administration, the mice in each group were intraperitoneally injected with naloxone at a dose of 15 mg/kg, and the number of jumps of each mouse within 15 minutes and body weight changes within 1 hour were observed. Results are shown in Table 4.

Table 4 Effects of drugs on mice with morphine dependence in jumping test

| Drug (mg/kg, po) | | Number of animals | Number of jumps | Body weight loss (g) |
|---|---|---|---|---|
| Blank control group | | 10 | 44.37±13.98 | 0.81±0.29 |
| Drug group 1 | Low dose | 10 | 44.11±14.02 | 0.80±0.20 |
| | Medium dose | 10 | 35.67±12.89* | 0.60±0.19* |
| | High dose | 10 | 32.64±12.78* | 0.57±0.21* |
| Drug group 2 | Low dose | 10 | 42.65±15.36 | 0.77±0.20 |
| | Medium dose | 10 | 31.81±14.01* | 0.53±0.30* |
| | High does | 10 | 29.89±13.63* | 0.50+0.19* |
| Drug group 3 | Low dose | 10 | 39.97±14.82 | 0.88±0.19 |
| | Medium dose | 10 | 34.87±13.78* | 0.56±0.24* |
| | High dose | 10 | 33.89+10.89* | 0.52+0.23* |
| Drug group 4 | Low dose | 10 | 43.28±12.40 | 0.78±0.26 |
| | Medium dose | 10 | 16.6±5.9** | 0.42±0.25** |
| | High dose | 10 | 11.9±7.0** | 0.32+0.21** |
| Drug group 5 | Low dose | 10 | 42.69±13.11 | 0.75±0.19 |
| | Medium dose | 10 | 15.9±4.8** | 0.40±0.19** |
| | High dose | 10 | 10.0±6.7** | 0.29±0.18** |

(continued)

| Drug (mg/kg, po) | | Number of animals | Number of jumps | Body weight loss (g) |
|---|---|---|---|---|
| Drug group 6 | Low dose | 10 | 41.86±11.99 | 0.76±0.21 |
| | Medium dose | 10 | 15.9±4.9** | 0.39±0.21** |
| | High dose | 10 | 11.6±6.3** | 0.29±0.19** |
| Drug group 7 | Low dose | 10 | 43.30±12.38 | 0.79±0.25 |
| | Medium dose | 10 | 17.2±6.1** | 0.43±0.27** |
| | High dose | 10 | 12.3±7.9** | 0.33±0.23** |
| Drug group 8 | Low dose | 10 | 43.01±12.99 | 0.75±0.19 |
| | Medium dose | 10 | 16.3±5.6** | 0.40±0.19** |
| | High dose | 10 | 11.9±6.7** | 0.28±0.18** |
| Drug group 9 | Low dose | 10 | 41.99±12.81 | 0.82±0.17 |
| | Medium dose | 10 | 16.0±4.7** | 0.38±0.17** |
| | High dose | 10 | 10.9±6.4** | 0.31±0.16** |
| Drug group 10 | Low dose | 10 | 42.99±13.11 | 0.79±0.20 |
| | Medium dose | 10 | 17.7±5.9** | 0.41±0.14** |
| | High dose | 10 | 12.9±5.4** | 0.29±0.17** |
| Drug group 11 | Low dose | 10 | 43.99±14.21 | 0.80±0.20 |
| | Medium dose | 10 | 34.12±11.21* | 0.70±0.18* |
| | High dose | 10 | 27.8±12.5* | 0.55±0.22* |
| Drug group 12 | Low dose | 10 | 44.59±14.61 | 0.83±0.31 |
| | Medium dose | 10 | 43.17±12.84 | 0.79±0.30 |
| | High dose | 10 | 44.28±14.01 | 0.82±0.29 |
| Note: compared with the blank control group, *P<0.5, indicating a significant difference, and *P<0.01, indicating an extremely significant difference. | | | | |

It can be seen from the foregoing table, after the mice administered with the compound tablets of the present disclosure are stimulated with naloxone, both the number of jumps and the body weight loss have significant differences (P<0.5) compared to the blank control group, which indicates that the compound tablets of the present disclosure have an effect of relieving withdrawal symptoms of the mice.

Moreover, after the mice intragastrically administered with the compound tablets of Examples 4 to 10 are stimulated with naloxone, both the number of jumps and the body weight loss have extremely significant differences (P<0.01) compared to the blank control group, which indicates that the compound tablets of the present disclosure have a better effect of relieving withdrawal symptoms of the mice with morphine dependence when a weight ratio of clonidine hydrochloride to tramadol hydrochloride is (0.01-0.56): 50.

(2) Rat stimulation test:

Rats were randomly divided into a blank control group and drug groups 1 to 12 according to the weight. The drug groups 1 to 12 (respectively administered with the compound tablets of Examples 1 to 10 and the tablets of Comparative Examples 1 and 2) were divided into low-dose groups, medium-dose groups, and high-dose groups, 15 rats per group. The rats in each group were intraperitoneally injected with morphine 3 times a day at administration doses of 10 mg/kg/time on day 1, 20 mg/kg/time on day 2 and day 3, and 30 mg/kg/time on day 4 to day 10. The rats in the blank control group and the drug groups were subjected to orally intragastric administration from day 8 of the test once a day (intragastric administration was performed 4.5 hours after the third intraperitoneal injection of morphine) for 3 consecutive days. An administration dose for the rats in the low-dose groups was 25 mg/kg, an administration dose for the rats in the medium-dose groups was 50 mg/kg, and an administration dose for the rats in the high-dose groups was 100 mg/kg. The rats in the blank control group were administered with an equal volume of normal saline. 5 hours after the last intraperitoneal injection of morphine, the rats were intraperitoneally injected with naloxone at a dose of 10 mg/kg, and withdrawal symptoms and body weight changes within 1 hour were observed. Results are shown in Table 5.

Table 5 Effects of drugs on rats with morphine dependence in stimulation test

| Drug (mg/kg, po) | | Number of animals | Withdrawal score | Body weight loss (g) |
|---|---|---|---|---|
| Blank control group | | 15 | 30.1+4.1 | 14.9±3.4 |
| Drug group 1 | Low dose | 15 | 29.6±3.9 | 14.6±3.0 |
| | Medium dose | 15 | 27.2±3.8 | 13.3±2.9 |
| | High dose | 15 | 18.2±2.9* | 12.1±3.1* |
| Drug group 2 | Low dose | 15 | 29.1+3.9 | 14.2+3.2 |
| | Medium dose | 15 | 26.3±4.0 | 12.6±2.9 |
| | High does | 15 | 17.4±2.8* | 11.8±2.8* |
| Drug group 3 | Low dose | 15 | 30.2+3.9 | 14.7±2.9 |
| | Medium dose | 15 | 28.6±3.2 | 13.9±3.1 |
| | High dose | 15 | 19.1±2.9* | 12.3±2.8* |
| Drug group 4 | Low dose | 15 | 14.98±5.4* | 10.2±3.0* |
| | Medium dose | 15 | 7.4±4.0** | 6.4±1.6** |
| | High dose | 15 | 4.3±1.8** | 3.9±1.8** |
| Drug group 5 | Low dose | 15 | 16.21±4.8* | 9.5±3.1* |
| | Medium dose | 15 | 8.8±4.1** | 5.9+1.5** |
| | High dose | 15 | 3.9±2.0** | 4.2±2.1** |
| Drug group 6 | Low dose | 15 | 14.63±5.6* | 10.6±2.6* |
| | Medium dose | 15 | 6.9±3.6** | 5.7±2.0** |
| | High dose | 15 | 4.4±2.0** | 4.3±2.2** |
| Drug group 7 | Low dose | 15 | 15.31±6.0* | 9.6±2.8* |
| | Medium dose | 15 | 7.1±3.9** | 6.1±1.7** |
| | High dose | 15 | 4.1±1.9** | 4.1±1.9** |
| Drug group 8 | Low dose | 15 | 14.86±5.9* | 10.1+3.3* |
| | Medium dose | 15 | 6.9±2.9** | 5.9±1.6** |
| | High dose | 15 | 3.9±2.1** | 4.0±1.7** |
| Drug group 9 | Low dose | 15 | 16.01±4.9* | 10.3±2.2* |
| | Medium dose | 15 | 7.3±3.2** | 5.7±1.8** |
| | High dose | 15 | 4.3±1.8** | 3.9±2.3** |
| Drug group 10 | Low dose | 15 | 16.02±5.3* | 9.8±2.6* |
| | Medium dose | 15 | 7.4±2.9** | 5.8±1.4** |
| | High dose | 15 | 3.8±1.7** | 3.8±1.9** |
| Drug group 11 | Low dose | 15 | 29.1±4.5 | 14.4±5.1 |
| | Medium dose | 15 | 22.2±5.2* | 12.5±3.1* |
| | High dose | 15 | 19.6±3.1* | 12.0±3.8* |

(continued)

| Drug (mg/kg, po) | | Number of animals | Withdrawal score | Body weight loss (g) |
|---|---|---|---|---|
| Drug group 12 | Low dose | 15 | 31.2±5.9 | 15.2±5.6 |
| | Medium dose | 15 | 29.8±4.9 | 14.6±4.8 |
| | High dose | 15 | 28.2±4.2 | 14.2±4.6 |
| Note: compared with the blank control group, *P<0.5, indicating a significant difference, and *P<0.01, indicating an extremely significant difference. | | | | |

It can be seen from the foregoing table that after producing morphine dependence, the rats in each group are stimulated with naloxone, and the results show that the rats in the blank control group have high irritability, diarrhea, salivation, gnashing, abnormal posture, and significant weight loss, and the withdrawal symptoms are obvious. However, after withdrawal of morphine, the rats administered with the compound tablets of Examples 1 to 10 of the present disclosure have mild tearing, diarrhea, salivation, and weight loss.

Compared with the blank control group, after producing morphine dependence and being simulated with naloxone, the rats intragastrically administered with the compound tablets of Examples 1 to 3 at the low dose and the medium dose do not have significant differences in withdrawal symptom scores and body weight loss, and the rats administered at the high dose have significant differences (P<0.5) in withdrawal symptom scores and body weight loss. The rats administered with the compound tablets of Examples 4 to 10 at the low dose have significant differences (P<0.5) in withdrawal symptom scores and body weight loss, and the rats administered at the medium dose and the high dose have extremely significant differences (P<0.01) in withdrawal symptom scores and body weight loss. The results show that the compound tablets of the present disclosure have an effect of alleviating withdrawal symptoms of the rats with morphine dependence. Moreover, the compound tablets of the present disclosure have a better effect of alleviating withdrawal symptoms of the rats with morphine dependence when a weight ratio of clonidine hydrochloride to tramadol hydrochloride is (0.01-0.56): 50.

Experimental Example III Addiction of tramadol and clonidine tablets and compound tablets

Rats weighing approximately 150 g, half male and half female, were taken and randomly divided into 10 groups, that is, a blank control group, a morphine group, a tramadol group, a clonidine group, and compound tablet groups 1 to 6 (the rats in the compound tablet group 1 were administered with the compound tablets of Example 1, the rats in the compound tablet group 2 were administered with the compound tablets of Example 2, the rats in the compound tablet group 3 were administered with the compound tablets of Example 3, the rats in the compound tablet group 4 were administered with the compound tablets of Example 6, the rats in the compound tablet group 5 were administered with the compound tablets of Example 7, and the rats in the compound tablet group 6 were administered with the compound tablets of Example 10).

The rats were administered 3 times a day for 30 days. Administration methods were as follows: (1) the blank control group: PO (oral administration); and 0 mg/kg/time; (2) the morphine group: ip (intraperitoneal injection); and 5 mg/kg/time in week 1, 10 mg/kg/time in week 2, 20 mg/kg/time in week 3, and 30 mg/kg/time from day 22 to day 30; (3) the tramadol group: PO; and 25mg/kg/time in week 1, 50 mg/kg/time in week 2, 100 mg/kg/time in week 3, and 200 mg/kg/time from day 22 to day 30; (4) the clonidine group: PO; and 0.075 mg/kg/time in week 1, 0.15 mg/kg/time in week 2, 0.3 mg/kg/time in week 3, and 0.6 mg/kg/time from day 22 to day 30; (5) the compound tablet groups: PO; and 25 mg/kg/time in week 1, 50 mg/kg/time in week 2, 100 mg/kg/time in week 3, and 200 mg/kg/time from day 22 to day 30. Withdrawal symptoms and body weight changes within one week after withdrawal of the rats in each group were observed. Results are shown in Table 6.

Table 6

| Drug (mg/kg, po) | Number of | animals Number of jumps | Body weight loss (g) |
|---|---|---|---|
| Blank control group | 6 | 4.1±1.5 | 2.1±0.8 |
| Morphine group | 6 | 20.1±2.1** | 10.1±2.1** |
| Tramadol group | 6 | 17.3±1.9** | 9.6±2.3** |
| Clonidine group | 6 | 4.2±1.3 | 1.9±0.5 |
| Compound tablet group 1 | 6 | 5.6±1.6 | 3.0±0.7 |

(continued)

| Drug (mg/kg, po) | Number of | animals Number of jumps | Body weight loss (g) |
|---|---|---|---|
| Compound tablet group 2 | 6 | 5.4±1.4 | 2.940.8 |
| Compound tablet group 3 | 6 | 5.9±1.5 | 3.2±0.9 |
| Compound tablet group 4 | 6 | 4.2±1.5 | 2.2±0.9 |
| Compound tablet group 5 | 6 | 4.0±1.4 | 2.0±0.7 |
| Compound tablet group 6 | 6 | 3.9±1.2 | 1.6±0.6 |
| Note: compared with the blank control group, *P<0.5, indicating a significant difference, and **P<0.01, indicating an extremely significant difference | | | |

It can be seen from the foregoing table that after the rats were orally administrated with the compound tablets for 30 consecutive days, compared with the blank control group, the rats in the compound tablet groups of the present disclosure have no significant differences in the number of jumps and body weight loss, and rats in the morphine group and the tramadol group have extremely significant differences (P<0.01). It indicates the compound tablets of the present disclosure do not produce withdrawal symptoms, and have low addiction and good safety.

It should be emphasized that the examples described in the present disclosure are illustrative rather than restrictive, so the present disclosure includes, but is not limited to, the examples described in the specific embodiments, and other embodiments obtained by those skilled in the art according to the technical solutions of the present disclosure shall also fall within the scope of protection of the present disclosure.

## Claims

1. A pharmaceutical composition for relieving or eliminating opioid withdrawal syndrome, comprising clonidine hydrochloride and tramadol hydrochloride,

   a mass ratio of clonidine hydrochloride to tramadol hydrochloride being (0.01-0.8): (40-60).

2. The pharmaceutical composition according to claim 1, wherein a total mass fraction of clonidine hydrochloride and tramadol hydrochloride in the pharmaceutical composition is 15-20%.

3. The pharmaceutical composition according to claim 2, wherein the mass ratio of clonidine hydrochloride to tramadol hydrochloride is (0.01-0.56): 50, and the total mass fraction of clonidine hydrochloride and tramadol hydrochloride in the pharmaceutical composition is 17-19%.

4. Application of the pharmaceutical composition according to any one of claims 1 to 3 in preparation of a medicine for relieving or eliminating opioid withdrawal syndrome.

5. A medicament prepared from the pharmaceutical composition according to any one of claims 1 to 3, composed of the pharmaceutical composition according to any one of claims 1 to 3 and pharmaceutically acceptable excipients.

6. The medicament according to claim 5, wherein the medicament comprises compound tablet, pill, granules, injection, powder, spray, sublingual tablet, capsule, and liquid preparation.

7. The medicament according to claim 6, wherein the medicament is a compound tablet, and the compound tablet comprises the following components in parts by weight: 0.01-0.8 parts of clonidine hydrochloride, 40-60 parts of tramadol hydrochloride, 110-120 parts of lactose hydrate, 100-120 parts of microcrystalline cellulose, 0.5-1 parts of silicon dioxide, and 2.0-3.6 parts of magnesium stearate.

8. The medicament according to claim 7, wherein the compound tablet comprises the following components in parts by weight: 0.01-0.56 parts of clonidine hydrochloride, 50 parts of tramadol hydrochloride, 114.5 parts of lactose hydrate, 112 parts of microcrystalline cellulose, 0.7 parts of silicon dioxide, and 2.8 parts of magnesium stearate.

9. The medicament according to claim 7 or 8, wherein a preparation method of the compound tablet comprises the following steps:

(1) dissolving a formulated amount of clonidine hydrochloride in water, granulating with a formulated amount of microcrystalline cellulose, sieving, and oven-drying to obtain granules; and

(2) mixing the granules obtained at step (1) with a formulated amount of tramadol hydrochloride and a formulated amount of lactose hydrate, adding a formulated amount of silicon dioxide and a formulated amount of magnesium stearate, mixing, compressing into tablets, and coating to obtain compound tablets.

10. Application of the medicament according to any one of claims 5 to 9 in preparation of a medicine for relieving or eliminating opioid withdrawal syndrome.

| INTERNATIONAL SEARCH REPORT | International application No. |
|---|---|
| | **PCT/CN2020/135368** |

**A. CLASSIFICATION OF SUBJECT MATTER**

A61K 31/4168(2006.01)i; A61K 31/137(2006.01)i; A61K 9/28(2006.01)i; A61K 47/38(2006.01)i; A61K 47/26(2006.01)i; A61K 47/04(2006.01)i; A61P 25/36(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, VEN(DWPI+SIPOABS), CNTXT, EPTXT, USTXT, WOTXT, 中国药物专利数据库, CNKI, 百度学术搜索, 超星读秀学术(CN), 万方(CN), Medline, ISI-WEB OF SCIENCE, EMBASE, CHEMICAL ABSTRACTS: 可乐定, 曲马多, clonidine, tramadol, Opioid, withdrawal, syndrome

**C. DOCUMENTS CONSIDERED TO BE RELEVANT**

| Category* | Citation of document, with indication, where appropriate, of the relevant passages | Relevant to claim No. |
|---|---|---|
| PX | CN 112494486 A (SHENZHEN SCIENCARE MEDICAL HEALTH INDUSTRY CO., LTD.) 16 March 2021 (2021-03-16) entire document | 1-10 |
| X | Ercan Gürses MD et al. "The addition of droperidol or clonidine to epidural tramadol shortens onset time and increases duration of postoperative analgesia" 《REGIONAL ANESTHESIA AND PAIN》 , Vol. 50, No. 2, 31 December 2003 (2003-12-31), see abstract, page 149 left-hand column paragraph 1 | 1-3, 5-9 |
| Y | CN 103495172 A (LU, Zhengtang) 08 January 2014 (2014-01-08) see claims 1, 3, 5 | 4, 10 |
| Y | Ercan Gürses MD et al. "The addition of droperidol or clonidine to epidural tramadol shortens onset time and increases duration of postoperative analgesia" 《REGIONAL ANESTHESIA AND PAIN》 , Vol. 50, No. 2, 31 December 2003 (2003-12-31), see abstract, page 149 left-hand column paragraph 1 | 4, 10 |
| A | CN 108030770 A (CHINA PHARMACEUTICAL UNIVERSITY) 15 May 2018 (2018-05-15) entire document | 1-10 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 August 2021** | **07 September 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2020/135368**

### C.   DOCUMENTS CONSIDERED TO BE RELEVANT

| Category* | Citation of document, with indication, where appropriate, of the relevant passages | Relevant to claim No. |
|---|---|---|
| A | CN 1679515 A (SUN, Yonghai) 12 October 2005 (2005-10-12)<br>entire document | 1-10 |

Form PCT/ISA/210 (second sheet) (January 2015)

15

**INTERNATIONAL SEARCH REPORT**
Information on patent family members

International application No.

**PCT/CN2020/135368**

| Patent document cited in search report | | | Publication date (day/month/year) | Patent family member(s) | Publication date (day/month/year) |
|---|---|---|---|---|---|
| CN | 112494486 | A | 16 March 2021 | None | |
| CN | 103495172 | A | 08 January 2014 | None | |
| CN | 108030770 | A | 15 May 2018 | None | |
| CN | 1679515 | A | 12 October 2005 | None | |

Form PCT/ISA/210 (patent family annex) (January 2015)

**REFERENCES CITED IN THE DESCRIPTION**

**Patent documents cited in the description**

- CN 103495172 A